# EUROPEAN PATENT SPECIFICATION

(11) **EP 3 225 239 B1**
(45) Date of publication and mention of the grant of the patent: **01.07.2020**
(21) Application number: 17161955.4
(22) Date of filing: 21.03.2017
(51) Int. Cl.: A61K 31/375, A61K 31/722, A61K 9/20, A23L 29/00, A23L 33/145

(54) **BODY WEIGHT CONTROL PREPARATION BASED ON CHITOSAN AND CELLULOSE**
ZUSAMMENSETZUNG AUF BASIS VON CHITOSAN UND ZELLULOSE ZUR KONTROLLE DES KÖRPERGEWICHTS
COMPOSITION À BASE DE CHITOSAN ET CELLULOSE POUR LE CONTRÔLE DU POIDS CORPOREL

(30) Priority: 23.03.2016 IT UA20161937
(43) Date of publication of application: 04.10.2017
(73) Proprietor: S.I.I.T. S.r.l.-Servizio Internazionale Imballaggi Termosaldanti, 20090 Trezzano sul Naviglio MI (IT)
(72) Inventor: MARCELLONI, Luciano, 20090 TREZZANO SUL NAVIGLIO (MI) (IT); COSTA, Andrea, 20090 TREZZANO SUL NAVIGLIO (MI) (IT)
(74) Representative: Bianchetti Bracco Minoja S.r.l.

(56) References cited:
- EP-A1- 2 865 278
- WO-A1-97/29760
- US-A- 5 453 282
- US-A1- 2002 164 352
- ZA-B- 200 106 447

## Description

### Field of invention

The invention relates to solid, oral compositions useful to control fat absorption in an individual.

### Background to the invention

Over a billion people worldwide are currently overweight, 20-30% of whom are obese. The incidence of obesity is also increasing among young people, and not only among those who live in the developed Western countries. The consequences of this situation will be significant growth in cardiovascular disease and chronic metabolic diseases like diabetes, and in costs for national health systems. There is consequently great interest in research into low-cost diet supplements of natural origin which can significantly contribute to preventing obesity.

Many useful products designed to control weight increase are currently based on chitosan.

Chitosan is an aminopolysaccharide which, due to the basic positively-charged amino groups present in its polysaccharide chain, is able to bind and sequester fatty acids in dietary lipid substances, thus mechanically preventing absorption of fats from food and, to a greater extent, reducing the absorption of dietary cholesterol by binding to bile acids and preventing the negatively-charged fats from emulsifying. This latter action also indirectly promotes the turnover and excretion of endogenous cholesterol. In view of these characteristics, chitosan is used in support treatments for weight loss or maintenance of the ideal weight.

Chitosan can be of animal or vegetable origin. It is obtained by synthesis from chitin, a constituent of the exoskeletons of many marine invertebrates and of the cell walls of fungi and yeasts. The procedure for its preparation involves hot basic hydrolysis of the acetylated amino groups present in chitin, followed by weakly acid and weakly basic washes. Processes for the preparation of chitosan from fungal or yeast biomasses are described in WO03/068824, US2007/0299034 and US2014/0220078. The structures of chitosan and chitin are reported in Figure 1.

For the purposes of the present invention, chitosan obtained from fungi or yeasts is indicated as vegetable chitosan or chitosan of vegetable origin.

Vegetable chitosan has a number of advantages, because the steps used in its extraction from vegetable sources leave lower heavy-metal residues. In fact, said steps can involve toxicity, albeit with a very low percentage, following long-term administration at the normally effective doses (0.5-2 g/day of chitosan). Moreover, it contains no trace of potential allergenic ingredients from crustacea, to which the population is increasingly allergic.

In the stomach and intestine, chitosan acts as a non-absorbable fibre, like cellulose.

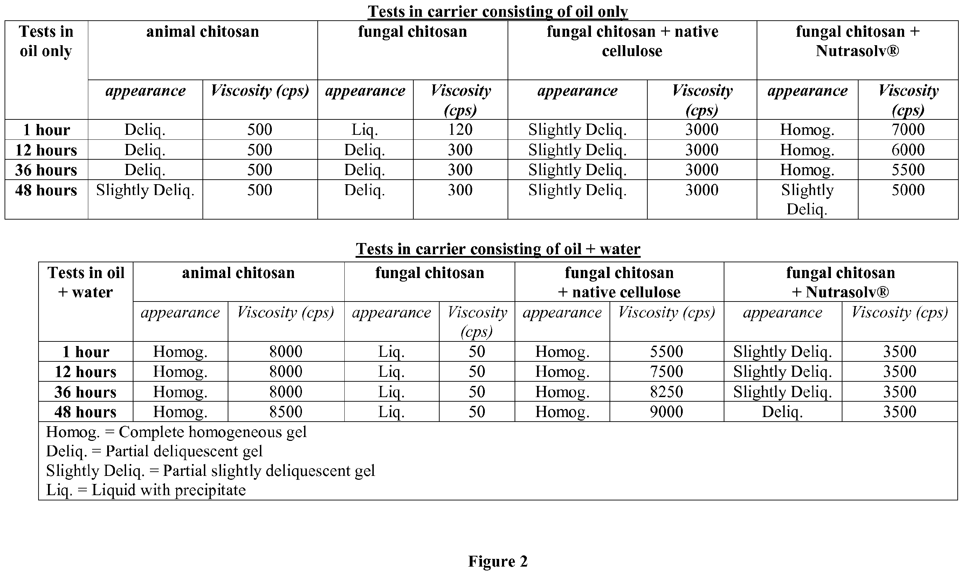

Chitosan of animal origin is an agent that increases the viscosity of foods, thus causing distension of the duodenum, and slows stomach voiding, reducing the sensation of hunger. The approximate viscosities for chitosan of animal and vegetable origin are reported in Figure 2.

Co-administration in a defined ratio with ascorbic acid (Vitamin C) can considerably improve the fat-sequestration effects of chitosan and stabilise the emulsions throughout the intestinal tract, without modifying its action mechanism.

Chitosan of vegetable origin may have a higher cost and show lower viscosity than that of animal origin, which renders the development of these forms less attractive.

Furthermore, it has been observed that in a medium which reproduces the gastrointestinal fluids, characterised by the presence of a mixture of water and oil, chitosan of vegetable origin may be less effective than chitosan of animal origin in binding fats.

It therefore became necessary to find a gelling component, preferably of vegetable origin, which, in combination with chitosan of vegetable origin, was able to reproduce the spontaneous gelling behaviour in water of chitosan of animal origin, and preferably also provided an absorption capacity compatible with oil absorption.

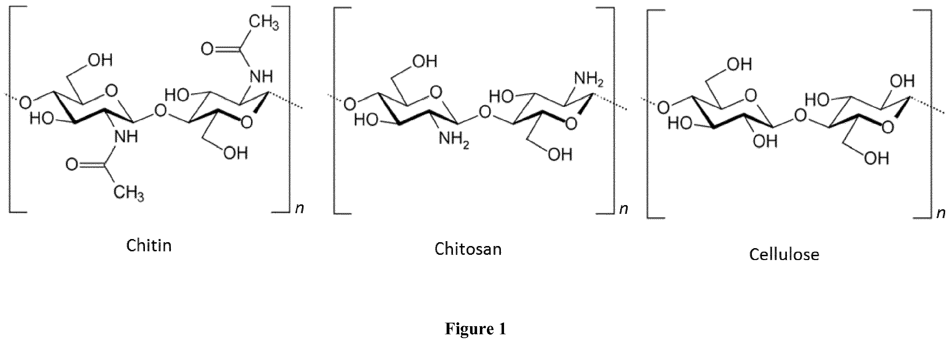

Cellulose is a polysaccharide consisting of glucose molecules (ranging from about 300 to 3,000 units) joined by a β(1→4) glycoside bond. Its structure is reported in Figure 1. It is obtained by purification and size reduction from alpha-cellulose in pulp extracted from fibrous vegetables. In powdered form it is used as a diluent in several oral forms. It has acceptable compression properties and is used as base material in powdered dose forms, and as suspension agent in aqueous suspensions for oral administration. Powdered cellulose presents as an odourless, tasteless white powder with varying particle sizes, ranging from a fine powder to a thick, coarse, soft, non-flowable granular powder. Said material is also suitable for wet granulation. It is practically insoluble in water, dilute acids and various organic solvents, although it disperses in most liquids.

Powdered cellulose is amorphous, and is distinguished from (micro)crystalline cellulose by the absence of crystalline zones where the polymer chains of this polysaccharide are aligned in parallel. In powdered cellulose, the polymer chains are tangled together.

Powdered cellulose, suitably purified, is used as a food additive, and is compatible with chitosan.

It has now surprisingly been found that powdered cellulose, combined with chitosan of vegetable origin, restores the spontaneous gelling properties in water and water/oil mixtures typical of chitosan of animal origin, and increases the direct fat-absorption properties of chitosan of vegetable origin.

### Description of figures

FIGURE 1. Formulas of chitin, chitosan and cellulose polysaccharides.
FIGURE 2. Ability to bind oil, demonstrated by analysis of the appearance (whether gelled or not) and viscosity of animal chitosan, fungal chitosan and mixtures of the latter with powdered cellulose or Nutrasolv® in cases wherein the carrier is oil alone or oil/water, after different contact times.
FIGURE 3. Representation of the SHIME® Prodigest system, consisting of five sequential reactors that simulate the different regions of the human gastro-intestinal tract.
FIGURE 4. Behaviour of the vegetable chitosan/powdered cellulose combination in the presence (left) and absence (right) of dietary lipids which are homogeneously emulsified.
FIGURE 5. Lipid binding by the fungal chitosan/powdered cellulose combination in ratios of about 1:3 compared with a non-lipid-binding control.
FIGURE 6. Lipid binding by chitosan of animal origin compared with a non-lipid-binding control.

### Description of the invention

The subject of the present invention is a solid, oral composition useful to control fat absorption in an individual, containing a combination of chitosan of vegetable origin and powdered native cellulose (powdered cellulose). A further subject of the invention is the use of said compositions to control obesity, excess weight and excess cholesterol and triglycerides.

The compositions according to the invention contain a combination of chitosan of vegetable origin and powdered native cellulose, and optionally ascorbic acid (vitamin C), together with excipients.

The chitosan which is preferably used for the purposes of the present invention is a compound corresponding to CAS number 9012-76-4 having the following characteristics:
- insoluble in water or ethanol but soluble in acidic and slightly acidic aqueous solutions;
- moisture content less than or equal to 10% by weight, measured by weight loss on drying test compared with the wet weight;
- degree of deacetylation exceeding 70% in mol %;
- 1% viscosity, in 1% acetic acid, (mPa.s) from 1 to 1000 cps depending on its molecular weight;
- molecular weight ranging from 10,000 Da to 2,000,000 Da;
- average particle size: over 95% less than 250 µm, particle size D 0.9 preferably less than or equal to 250 µm (particle-size analysis with laser diffraction, Mastersizer 2000, ISO 13320);
- compacted density ranging between 0.7 and 1.0 g/cm³ (measured according to European Pharmacopoeia EP 2.9.34).

The most common names of this material are vegetable chitosan, non-animal chitosan, and fungal chitosan (due to the extractive origin of its precursor chitin).

Chitosan preferably has an average molecular weight ranging between 10,000 and 1,500,000 daltons, even more preferably between 10,000 and 500,000 daltons, and a degree of deacetylation ranging between 85% and 99%.

The chitosan according to the present invention is preferably obtained from fungal biomasses.

The cellulose which is preferably used for the purposes of the present invention is powdered native cellulose (native cellulose or powdered cellulose) corresponding to CAS number 9004-34-6. The most common names of this material are powdered cellulose (British, Japanese and European pharmacopoeias) and *Cellulosi pulvis* (US pharmacopoeia). Its tradenames are Arbocel®; E460; Elcema; Sanacel; and Solka-Floc.

The cellulose described in the Handbook of Pharmaceutical Excipients, fifth edition, monograph "Cellulose, powdered", having the following characteristics, is more preferred: average molecular weight 243,000 Da, average particle size ranging between 60 µm and 200 µm, preferably 200 µm. The following are the particle sizes of samples of powdered cellulose usable for the purposes of the present invention:
Arbocel® M80: average particle size 60 µm;
Arbocel® P 290: average particle size 70 µm;
Arbocel® A 300: average particle size: 200 µm.

The weight ratios between vegetable chitosan and powdered cellulose range between 1:1 and 1:5, preferably between 1:2 and 1:4, and more preferably 1:3.

Said ratios are advantageous as regards fat-absorption efficacy and to reduce the final cost of chitosan of vegetable origin. The presence of a quantity of vegetable chitosan exceeding said ratios not only reduces the swelling capacity, but also the viscosity of cellulose.

A 1:3 quantitative ratio of vegetable chitosan : powdered cellulose has proved ideal. The viscosity of the combination obtained with said proportions is similar to that of animal chitosan.

The compositions according to the invention can optionally contain ascorbic acid.

After thorough mixing, the vegetable chitosan/powdered cellulose combination acts as a single swellable but not absorbable fibre, which binds larger quantities of lipid substances than vegetable chitosan alone, and increases the spontaneous gelling capacity of vegetable chitosan to a level equal to or greater than that of animal chitosan.

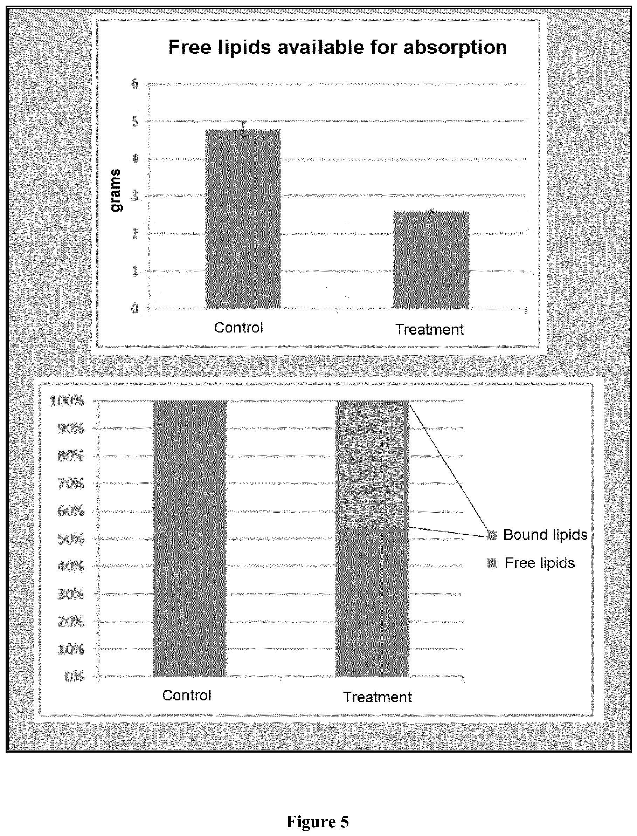

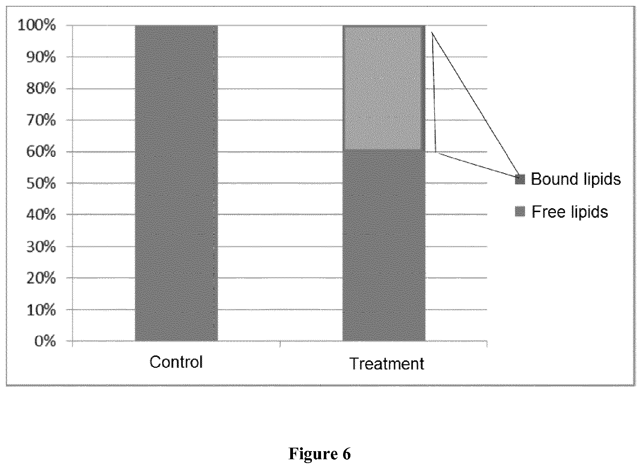

In laboratory tests conducted under simulated gastrointestinal conditions, the vegetable chitosan/powdered cellulose combination significantly reduced (by about 45%) the proportion of fats potentially available for absorption, as illustrated in Example 4 and Figure 5. This result is better than that obtainable with chitosan of animal origin alone, which results in about 40% of fats potentially available for absorption (Figure 6).

The maximum quantities per meal which are useful to lose 2-3% of body mass in a 2-month treatment period are those contained in four tablets taken before every meal, each containing 600 mg of the vegetable chitosan/powdered cellulose combination.

One dosage unit generally contains:
70 to 300 mg (preferably 140 mg) of fungal chitosan;
150 to 800 mg (preferably 460 mg) of powdered cellulose;
optionally, 14 to 100 mg (preferably 50 mg) of ascorbic acid.

The quantity can vary depending on the form of administration, which can be tablets, sachets, cachets, stickpacks, or any solid form designed for oral use in general. The composition may further contain excipients and other components suitable for pharmaceutical or dietary applications.

The compositions according to the invention can be obtained by thoroughly dry-mixing chitosan, cellulose and optionally ascorbic acid with suitable excipients, or alternatively by a common wet granulation process. This can be conveniently followed by compression into bars, chewable tablets or tablets, or by batching to fill sachets after mixing with glidants, sweeteners and flavourings.

To guarantee maximum efficacy in the two different action sites of chitosan (gastric and intestinal), the tablets can consist of two or three layers with different release kinetics or different compositions.

The common techniques used to prepare mixtures intended to undergo direct compression or granulation of some of the ingredients and subsequent mixing with powder lubricants and glidants can be conveniently used in the process for preparation of the mixture of ingredients.

A particular embodiment of the invention consists of a two-layer tablet: a "rapid" layer for immediate release and a "delayed" layer for delayed intestinal release.

The process for preparation of the two-layer tablet according to the invention is the one commonly used in tablet preparation, employing machines for granulation and drying of the ingredients, mixers and tablet presses, the latter being of a type suitable for the preparation of multi-layer tablets.

The mixing stages are followed by compression in particular rotary tablet presses able to generate tablets with multiple superimposed layers.

Said machines are typically fitted with at least two loading hoppers for the different mixtures corresponding to the different layers, and at least two compression stations, one after the other on the same rotor, where the two different mixtures to be compressed are batched.

In a typical preparation, a first hopper discharges the quantities of the mixture of one of the two layers to be compressed, and the mixture is compressed in a first compression station. The half tablet obtained is then conveyed to the next compression station. There, it is coated by the second granulate batched by the second hopper, which is directly compressed onto the first layer, thus giving rise to the two-layer tablet.

The invention will now be further illustrated by the following examples.

### EXAMPLES

### Example 1 - Three-layer rapid-release tablet containing the vegetable chitosan/powdered cellulose combination

Composition with simethicone and *Phaseolus vulgaris* dried extract separate from the chitosan/cellulose mixture:

| | **mg/tablet** |
|---|---|
| **Outer layer 1** | |
| Fungal chitosan | 70.000 |
| Powdered cellulose | 230.000 |
| Dicalcium phosphate | 103.608 |
| Vitamin C | 17.692 |
| Red iron oxide | 2.200 |
| PVP XL | 2.000 |
| Syloid | 4.000 |
| Glyceryl behenate | 6.000 |
| Hydroxypropylcellulo se | 6.000 |
| Veg. magnesium stearate | 5.000 |
| Croscarmellose sodium | 3.500 |

| **Middle layer 2** | |
|---|---|
| Simethicone | 25.000 |
| Silica | 25.000 |
| M alto dextrins | 25.000 |
| Kidney bean (Phaseolus vulgaris) dried extract | 25.000 |
| Vitamin B2 (riboflavin-based) | 1.200 |
| Microcrystalline cellulose | 76.500 |

| **Middle layer 2** | |
|---|---|
| M alto dextrins | 3.300 |
| Levilite | 3.000 |
| Talc | 3.000 |
| Hydroxypropyl methylcellulose | 23.000 |

| **Outer layer 3** | |
|---|---|
| Fungal chitosan | 70.000 |
| Powdered cellulose | 230.000 |
| Dicalcium phosphate | 103.608 |
| Vitamin C | 17.692 |
| Red iron oxide | 2.200 |
| PVP XL | 2.000 |
| Silica | 4.000 |
| Glyceryl behenate | 6.000 |

| | **mg/tablet** |
|---|---|
| **Outer layer 3** | |
| Hydroxypropylcellulose | 6.000 |
| Veg. magnesium stearate | 5.000 |
| Croscarmellose sodium | 3.500 |
| **TOTAL** | **1110.00** |

### Example 2 - Two-layer tablet, one of which providing delayed release of the vegetable chitosan/powdered cellulose combination

Same ingredients with differentiated rapid/delayed release:

| | **mg/tablet** |
|---|---|
| **Immediate-release layer** | |
| Fungal chitosan | 90.000 |
| Powdered cellulose | 210.000 |
| Dicalcium phosphate | 103.608 |
| Vitamin C | 17.692 |
| Red iron oxide | 2.200 |
| PVP XL | 2.000 |
| Syloid | 4.000 |
| Glyceryl behenate | 6.000 |
| Hydroxypropylcellulo se | 6.000 |
| Veg. magnesium stearate | 5.000 |
| Croscarmellose sodium | 3.500 |

| **Delayed-release layer** | |
|---|---|
| Fungal chitosan | 90.000 |
| Powdered cellulose | 210.000 |
| Dicalcium phosphate | 93.095 |
| Granular calcium carbonate | 155.513 |
| Vitamin C | 17.692 |
| YELLOW iron | 2.200 |
| PVP XL | 2.000 |
| Silica | 4.000 |
| Glyceryl behenate | 6.000 |
| Hydroxypropyl methylcellulose | 55.000 |
| Hydroxypropylcellulose | 6.000 |
| Veg. magnesium stearate | 5.000 |
| Croscarmellose sodium | 3.500 |
| **TOTAL** | **1100.00** |

It should be noted that in this last composition, in view of the particular avidity of water for native cellulose, the presence of the two disintegrating agents PVP XL and croscarmellose sodium was also maintained in the delayed-release layer. This allows complete penetration of water or biological fluids so that the polymer with hydroxypropyl methylcellulose matrix in the delayed-release layer is completely hydrated.

This particular formulation as a "double-layer" tablet is designed to ensure a better action through the gastrointestinal tract, and was studied with the *in vitro* test described in example 5 to evaluate the performance of the fungal chitosan/powdered cellulose/ascorbic acid combination by comparison with the animal chitosan/ascorbic acid combination.

### EXAMPLE 3 - Sachets for reconstitution in water

| | **mg/sachet** |
|---|---|
| Fungal chitosan | 300.000 |
| Powdered cellulose | 800.000 |
| Trehalose | 1500.000 |
| Vitamin C | 47.692 |
| Orange flavouring | 6.200 |
| Sodium carboxymethylcellulose | 4.000 |
| Silica | 4.000 |

### Example 4 - Physical tests

Comparative physical laboratory tests were conducted with the following products:
1) chitosan of animal origin from prawn shells (high-molecular-weight chitosan, > 800,000 Da, over 90% deacetylated);
2) fungal chitosan (molecular weight ranging between 10,000 and 500,000 Da, at least 70% deacetylated);
3) the fungal chitosan mentioned in the preceding paragraph mixed with Arbocel 300® powdered cellulose in the ratio of 1:3;
4) fungal chitosan as above, mixed with a coprocessed microcrystalline cellulose material functionalised with silica (Nutrasolv 90/F ®), a potent aspecific liquid absorbent, in the ratio of 1:3.

The characteristics of the two celluloses used are described in the Handbook of Pharmaceutical Excipients, in the monographs Powdered Cellulose and Silicified Cellulose.

The materials were immersed in liquids with different levels of hydrophilia:
a) oil alone (sunflower seed oil); non-hydrophilic, to maximise the fat-binding capacity;
b) mixture of 50% water and 50% sunflower seed oil, commonly present in a normal diet; partly hydrophilic, to simulate a real meal that contains both lipids and water.

The mixtures of the two polysaccharides were mixed and evenly dispersed in liquids a) and b) and observed at the starting time and after 12, 36 and 48 hours for appearance and viscosity, the latter being measured with a Brookfield apparatus, operated at 10 rpm with spindle no. 3.

A maximum contact time with the gastroenteric tract was simulated, including an average intestinal transit time in humans.

A homogeneous mixture that generates a uniform gel without deliquescence for up to 72 hours was considered to be a good result which can be observed visually, and favours physical absorption of fats.

The results are shown in Figure 2.

The tests indicate that the vegetable chitosan/powdered cellulose mixture in the partly hydrophilic mixture, ie. in a simulation of a meal + digestive fluids, provides a result very similar to, or even better than, that obtained with animal chitosan.

It was found that the combination with native cellulose, which in oil alone is inferior to Nutrasolv® in binding fats, becomes very effective with oil/water, exceeding the binding power of Nutrasolv® in terms of duration, with behaviour similar to that of chitosan of animal origin.

### Example 5 - Simulated digestion test

The vegetable chitosan/powdered cellulose combination is a slimming adjuvant based on the synergy of three different active ingredients: chitosan of fungal origin, ascorbic acid and powdered cellulose (Arbocel® A300), able to reduce the absorption of dietary lipids. This *in vitro* study was conducted to investigate the action mechanism and efficacy of double-layer tablets containing the vegetable chitosan/powdered cellulose combination in the upper human gastrointestinal tract.

The study involved:
- simulating the physiological conditions in the upper gastrointestinal tract;
- measuring the fraction of lipids bound to the vegetable chitosan/powdered cellulose combination after passing through the upper gastrointestinal tract.

### In vitro model of gastrointestinal tract with simulated digestion.

*In vitro* approaches to the study of the gastrointestinal tract and intestinal microbial processes provide an experimental method for studying the action mechanism of specific functional ingredients. It is now recognised that the application of well-designed continuous models allows in-depth study of the biological/mechanical activity of selected molecules in the intestine under representative environmental conditions. These concepts were used to study the action mechanism of the vegetable chitosan/powdered cellulose combination in the simulated upper human gastrointestinal tract, and the ability of said combination to inhibit the absorption of dietary lipids.

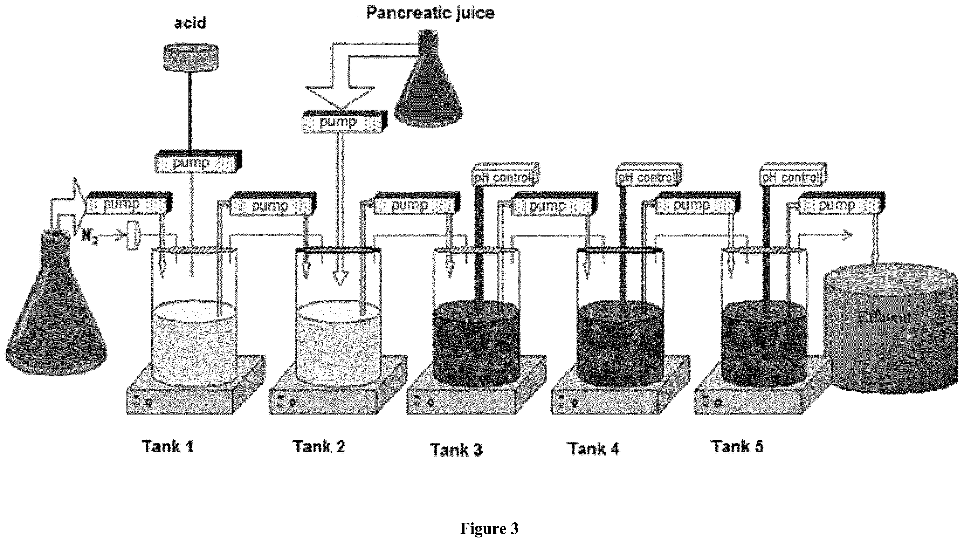

The study was conducted in a reactor configuration that operates as simulator of the human microbial ecosystem, consisting of a succession of five reactors that simulate the conditions existing in the various parts of the human gastrointestinal tract (Figure 3). The test samples containing nutrients and the fungal chitosan/powdered cellulose combination undergo all the various stages of food absorption and digestion, after specified time intervals. The first two reactors simulate the stomach (VI) and duodenum (V2), at a low pH and in the presence of digestive enzymes and bile acids. Enzymatic degradation and the absorption processes that take place in the small and large intestines are simulated in reactors V3, V4 and V5. The total residence time in the last three vessels, simulating the large intestine, is 72 hours. The absorption processes that take place in the small intestine are simulated by dialysis tubes. However, in this study, attention focuses solely on the upper gastrointestinal tract, simulating the physiological action of the stomach and small intestine, without considering the microflora of the colon.

The tests were conducted with a double-layer tablet containing the fungal chitosan/powdered cellulose combination, consisting of a rapid-release (gastric release) stage and a delayed-release (duodenal release) stage, as described in Example 2. In the stomach and intestine, the vegetable chitosan/powdered cellulose combination behaves like a soluble but non-absorbable fibre able to bind and sequester dietary lipid substances. In this way, it mechanically inhibits the absorption of dietary fats.

After simulated gastrointestinal transit at pH 4.2-1.8; in the presence of pepsin and mucus; for 90 minutes' incubation, after which the pH was increased to 6.8; in the presence of 12.5 g/L bicarbonate and 0.9 g/L pancreatin (a mixture representative of the enzymes secreted by the pancreas) for 180 minutes, the fraction of the vegetable chitosan/powdered cellulose combination bound to the lipids was separated from the liquid fraction by simple gravity, dried and weighed.

In order to obtain a reliable reference to interpret the data, two simulations were used:
- test of the tablet containing the vegetable chitosan/powdered cellulose combination with/without the addition of lipids; this test evaluated the behaviour of chitosan under representative gastrointestinal conditions;
- at the end of the simulation of transit through the small intestine, the fluids were centrifuged, and the fraction of the vegetable chitosan/powdered cellulose combination bound to the lipids (corresponding to the remaining quantity of unabsorbed lipids) was separated from the liquid fraction. This allowed evaluation of the quantity of dietary lipids that remain in the system and are available for absorption, in the presence of the fungal chitosan/powdered cellulose combination.

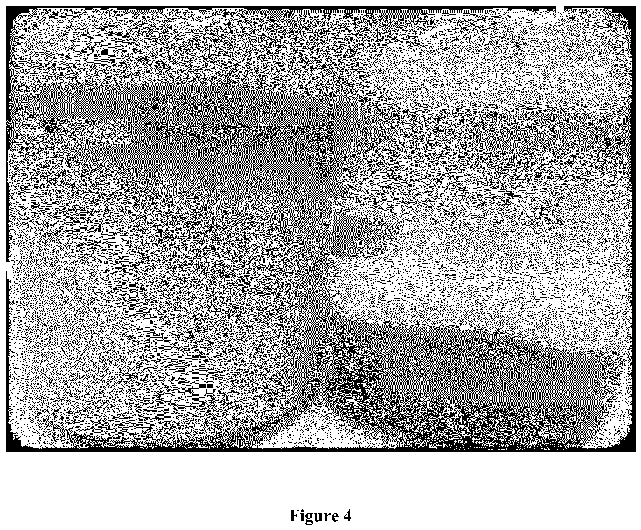

When the fungal chitosan/powdered cellulose combination is in the presence of fat, it forms a floating layer on the surface of the digestive fluid, whereas in the absence of oil, it sinks to the bottom of the beaker (Figure 4).

Figure 5 shows the quantity of lipids potentially available for absorption at the end of the small intestine simulation. In the presence of the vegetable chitosan/powdered cellulose combination, the fraction of lipids potentially available for absorption is reduced by about 45.8% (p <0.01 according to Student's t-test). For a better portrayal of the effect of the product, the same data can be displayed as the percentage of free lipids and lipids bound to the vegetable chitosan/powdered cellulose combination.

Results superior to those obtained with the same quantities of chitosan of animal origin, equal to the sum of the powdered cellulose and vegetable chitosan, in the presence of vitamin C, were obtained under the same experimental conditions (Figure 6).

## Claims

1. A solid, oral composition containing a combination of chitosan from fungi or yeasts, powdered amorphous cellulose and optionally excipients.

2. The composition of claim 1, further containing ascorbic acid.

3. The composition of claims 1 and 2, wherein the chitosan has the following characteristics:
- deacetylation degree higher than 70% (mol%)
- molecular weight ranging from 10,000 Da to 2,000,000 Da.

4. The composition of claim 3, wherein the chitosan has the following characteristics:
- molecular weight ranging from 10,000 to 1,500,000 Da, preferably from 10,000 to 500,000 Da;
- deacetylation degree 85% to 99%.

5. The composition according to any one of claims 1-4, wherein the powdered cellulose has an average particle size ranging from 60 µm to 200 µm.

6. The composition according to any one of claims 1-4, wherein the powdered cellulose has an average molecular weight of 243,000 Da.

7. The composition according to any one of claims 1-6, wherein the respective amounts of chitosan and powdered cellulose range from 1:1 to 1:5, preferably from 1:2 to 1:4, more preferably 1:3.

8. The composition according to any one of claims 1-7, containing, in a dosage unit:
from 70 to 300 mg chitosan;
from 150 to 800 mg powdered cellulose;
and optionally from 14 to 100 mg ascorbic acid.

9. The composition of claim 8, containing, in a dosage unit:
140 mg chitosan;
460 mg powdered cellulose;
and, optionally, 50 mg ascorbic acid.

10. The composition of claims 1-9, which is in the form of a tablet, sachet, cachet or stickpack.

11. The composition of claim 10, which is in the form of a multi-layered tablet.

12. A composition according to any one of claims 1-11, for use in controlling obesity, excess weight, and excess cholesterol and triglycerides.

## Patentansprüche

1. Feste orale Zusammensetzung, die eine Kombination von Chitosan aus Pilzen oder Hefen, pulverisierter amorpher Cellulose und gegebenenfalls Exzipienten enthält.

2. Zusammensetzung nach Anspruch 1, die ferner Ascorbinsäure enthält.

3. Zusammensetzung nach Anspruch 1 und 2, wobei das Chitosan die folgenden Eigenschaften aufweist:
- Deacetylierungsgrad höher als 70% (Mol-%)
- Molekulargewicht im Bereich von 10.000 Da bis 2.000.000 Da.

4. Zusammensetzung nach Anspruch 3, wobei das Chitosan die folgenden Eigenschaften aufweist:
- Molekulargewicht im Bereich von 10.000 bis 1.500.000 Da, vorzugsweise von 10.000 bis 500.000 Da;
- Deacetylierungsgrad 85% bis 99%.

5. Zusammensetzung nach einem der Ansprüche 1 bis 4, wobei die pulverisierte Cellulose eine durchschnittliche Partikelgröße im Bereich von 60 µm bis 200 µm aufweist.

6. Zusammensetzung nach einem der Ansprüche 1 bis 4, wobei die pulverisierte Cellulose ein durchschnittliches Molekulargewicht von 243.000 Da aufweist.

7. Zusammensetzung nach einem der Ansprüche 1 bis 6, wobei die jeweiligen Mengen an Chitosan und pulverisierter Cellulose im Bereich von 1:1 bis 1:5, vorzugsweise von 1:2 bis 1:4, bevorzugter 1:3 liegen.

8. Zusammensetzung nach einem der Ansprüche 1 bis 7, die in einer Dosierungseinheit enthält:
70 bis 300 mg Chitosan;
150 bis 800 mg pulverisierte Cellulose;
und gegebenenfalls 14 bis 100 mg Ascorbinsäure.

9. Zusammensetzung nach Anspruch 8, die in einer Dosierungseinheit enthält:
140 mg Chitosan;
460 mg pulverisierte Cellulose;
und gegebenenfalls 50 mg Ascorbinsäure.

10. Zusammensetzung nach den Ansprüchen 1 bis 9, die in Form einer Tablette, eines Sachets, eines Cachets oder eines StickPacks vorliegt.

11. Zusammensetzung nach Anspruch 10, die in Form einer mehrschichtigen Tablette vorliegt.

12. Zusammensetzung nach einem der Ansprüche 1 bis 11 zur Verwendung bei der Kontrolle von Adipositas, Übergewicht und überschüssigem Cholesterin und überschüssigen Triglyceriden.

## Revendications

1. Composition solide destinée à une administration par voie orale qui contient une combinaison de chitosane que l'on obtient à partir de champignons ou de levures, de cellulose amorphe pulvérulente et, de manière facultative, d'excipients.

2. Composition selon la revendication 1, qui contient en outre de l'acide ascorbique.

3. Composition selon les revendications 1 et 2, dans laquelle le chitosane possède les caractéristiques suivantes :
- un degré de désacétylation supérieur à 70 % (moles %) ;
- un poids moléculaire qui se situe dans la plage de 10.000 Da. à 2.000.000 Da.

4. Composition selon la revendication 3, dans laquelle le chitosane possède les caractéristiques suivantes :
- un poids moléculaire qui se situe dans la plage de 10.000 à 1.500.000 Da, de préférence dans la plage de 10.000 à 500.000 Da;
- un degré de désacétylation de 85 % à 99 %.

5. Composition selon l'une quelconque des revendications 1 à 4, dans laquelle la cellulose pulvérulente possède une granulométrie moyenne qui se situe dans la plage de 60 µm à 200 µm.

6. Composition selon l'une quelconque des revendications 1 à 4, dans laquelle la cellulose pulvérulente possède un poids moléculaire moyen de 243.000 Da.

7. Composition selon l'une quelconque des revendications 1 à 6, dans laquelle les quantités respectives de chitosane et de cellulose pulvérulente se situent dans une plage de 1:1 à 1:5, de préférence de 1:2 à 1:4, de manière plus préférée s'élèvent à 1:3.

8. Composition selon l'une quelconque des revendications 1 à 7, qui contient, dans une unité posologique :
de 70 à 300 mg de chitosane ;
de 150 à 800 mg de cellulose pulvérulente ;
et, de manière facultative, de 14 à 100 mg d'acide ascorbique.

9. Composition selon la revendication 8, qui contient, dans une unité posologique :
140 mg de chitosane ;
460 mg de cellulose pulvérulente ;
et, de manière facultative, 50 mg d'acide ascorbique.

10. Composition selon l'une quelconque des revendications 1 à 9, qui se présente sous la forme d'un comprimé, d'un sachet, d'un cachet ou d'un sachet tubulaire.

11. Composition selon la revendication 10, qui se présente sous la forme d'un comprimé multicouche.

12. Composition selon l'une quelconque des revendications 1 à 11, pour son utilisation dans la lutte contre l'obésité, contre un excès de poids et contre un excès de cholestérol et de triglycérides.
